# EUROPÄISCHE PATENTSCHRIFT

(11) **EP 0 595 942 B1**
(45) Veröffentlichungstag und Bekanntmachung des Hinweises auf die Patenterteilung: **28.02.1996**
(21) Anmeldenummer: 92916018.2
(22) Anmeldetag: 16.07.1992
(51) Int. Cl.: C08G 65/34, C08G 65/48, C07C 41/09, C08G 18/48

(54) **VERFAHREN ZUR HERSTELLUNG VON POLYOLVERBINDUNGEN**
METHOD OF PREPARING POLYOL COMPOUNDS
PROCEDE DE FABRICATION DE COMPOSES POLYOLS

(30) Priorität: 25.07.1991 DE 4124665
(43) Veröffentlichungstag der Anmeldung: 11.05.1994
(73) Patentinhaber: Henkel Kommanditgesellschaft auf Aktien, D-40191 Düsseldorf (DE)
(72) Erfinder: KLEIN, Johann, D-4000 Düsselfdorf 1 (DE); DAUTE, Peter, D-4300 Essen 1 (DE); HEES, Udo, D-5440 Mayen 1 (DE); BEUER, Bernd, D-4019 Monheim 2 (DE)
(86) Internationale Anmeldenummer: EP9201617
(87) Internationale Veröffentlichungsnummer: WO9302124

(56) Entgegenhaltungen:
- EP-A- 0 076 682
- DE-A- 3 446 720
- US-A- 2 772 245
- US-A- 3 045 034
- PATENT ABSTRACTS OF JAPAN vol. 13, no. 372 (C-627)(3720) 17. September 1989
- DATABASE WPIL Derwent Publications Ltd., London, GB; AN 83-835993(49)

## Beschreibung

### Gebiet der Erfindung

Die Erfindung betrifft ein Verfahren zur Herstellung von Polyolverbindungen durch Kondensation von Glycerin in Gegenwart von Lithiumverbindungen und gegebenenfalls nachfolgender Umesterung der erhaltenen Oligoglyceringemische mit Fettsäureglyceridestern sowie deren Verwendung zur Herstellung von Polymeren.

### Stand der Technik

Zur Herstellung von Polyurethanmassen werden Diisocyanate mit Stoffen umgesetzt, die mindestens zwei freie Hydroxylgruppen aufweisen. Als Polyolverbindungen für die Herstellung derartiger Kunststoffe kommen beispielsweise Ringöffnungsprodukte von Epoxidverbindungen mit Alkoholen in Betracht [**Fette, Seifen, Anstrichmitt., 89, 147 (1987)**]. Auch Oligoglyceringemische sind für diesen Anwendungszweck prinzipiell geeignet. Da die Eigenkondensation des Glycerins jedoch üblicherweise in Gegenwart von starken Basen, insbesondere in Gegenwart von Natrium- oder Kaliumhydroxid, durchgeführt wird, enthalten die resultierenden technischen Oligoglyceringemische in der Regel einen hohen Restgehalt an Alkali, der in der Polyurethanreaktion äußerst störend wirkt und zu einem unerwünschten Schrumpfen der Kunststoffmassen führen kann. Auch die weitere Umsetzung der Oligoglyceringemische mit Fetten und Ölen zu sogenannten Umesterungspolyolen oder Uralkyden, die sich ebenfalls als Rohstoffe für die Herstellung von Polyurethanen eignen, ist aus dem gleichen Grunde problematisch. Nach dem bisherigen Stand der Technik ist es daher erforderich, Oligoglyceringemische vor einer Weiterreaktion mit hohem technischen Aufwand zu entsalzen.

Die Aufgabe der Erfindung bestand nun darin, ein neues Verfahren zur Herstellung von Polyolen auf Basis von Eigenkondensationsprodukten des Glycerins zur Verfügung zu stellen, das frei von den geschilderten Nachteilen ist.

### Beschreibung der Erfindung

Gegenstand der Erfindung ist ein Verfahren zur Herstellung von Polyolverbindungen, das sich dadurch auszeichnet, daß man
a) Glycerin in Gegenwart von 0,001-0,1 Gew.%-bezugen auf das Glycerin Lithiumhydroxid, oder Lithiumseifen von gegebenenfalls hydroxysubstituierten Fettsäuren mit 6 bis 22 Kohlenstoffatomen und 0,1,2 oder 3 Doppelbindungen kondensiert
b) das Reaktionswasser kontinuierlich abdestilliert und
c) das erhaltene Oligoglyceringemisch gegebenenfalls mit Fettsäureglyceridestern einer Umesterung unterwirft.

Überraschenderweise wurde gefunden, daß die an sich bekannte Eigenkondensation des Glycerins auch mit äußerst geringen Katalysatormengen durchgeführt werden kann, wenn man als Katalysatoren Lithiumverbindungen, insbesondere Lithiumhydroxid verwendet. Die Erfindung beruht auf der Erkenntnis, daß die Alkalikonzentration in den nach dem erfindungsgemäßen Verfahren hergestellten Oligoglyceringemischen so gering ist, daß weder eine Beeinträchtigung der Polyurethanreaktion, noch der daraus resultierenden Kunststoffmassen erfolgt. Die Erfindung schließt darüber hinaus die Erkenntnis ein, daß der Gehalt an Lithiumverbindungen in den Oligoglyceringemischen ausreichend ist, um eine nachfolgende Umesterung mit Fettsäureglyceriden zu katalysieren, so daß der Einsatz eines zusätzlichen Umesterungskatalysators nicht erforderlich wird.

Als **Lithiumverbindungen** kommen neben Lithiumhydroxid auch Lithiumseifen, also Salze des Lithiums mit gegebenenfalls hydroxyfunktionalisierten Fettsäuren mit 6 bis 22 Kohlenstoffatomen und 0, 1, 2 oder 3 Doppelbindungen in Betracht. Typische Beispiele sind die Lithiumsalze der Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, 12-Hydroxystearinsäure, Ricinolsäure, Arachinsäure, Gadoleinsäure, Behensäure oder Erucasäure. Lithiumsalze ungesättigter Fettsäuren sind bei Normaltemperatur flüssig und deshalb leicht dosierbar; sie werden daher vorzugsweise verwendet. Die Lithiumseifen können der Reaktionsmischung direkt zugesetzt werden; sie können sich jedoch auch in situ erzeugt werden, beispielsweise aus Lithiumhydroxid und einer Fettsäure oder einem Fettsäureester.

Die **Einsatzmenge der Lithiumverbindungen** beträgt 0,001 bis 0,1, vorzugsweise 0,002 bis 0,05 und insbesondere 0,005 bis 0,01 Gew.-% - bezogen auf das Glycerin. Da Lithiumionen mit den in Gläsern enthaltenen Alkaliionen austauschen können, was zu einer Verringerung der Konzentration an Lithiumionen führen kann, empfiehlt es sich, das Verfahren in Geräten aus Stahl oder ähnlich inerten Matrialien durchzuführen.

Zur **Durchführung der Kondensationsreaktion** können Glycerin und Lithiumverbindungen vorgelegt und in einer Inertgasatmosphäre auf Temperaturen von 200 bis 300, vorzugsweise 220 bis 280°C erhitzt werden. Zur Verlagerung des Gleichgewichtes empfiehlt es sich, das entstehende Kondensationswasser beispielsweise über einen Wasserabscheider abzutrennen.

Wird die Herstellung eines Oligoglyceringemisches mit hohem Diglyceringehalt angestrebt, ist es vorteilhaft, die Kondensation abzubrechen, sobald die zur Bildung von Diglycerin theoretisch erforderliche Menge Wasser abgeschieden worden ist. Im allgemeinen beträgt die Reaktionszeit 1 bis 30, vorzugsweise 3 bis 15 h. Falls gewünscht, kann das Diglycerin aus dem gebildeten Oligoglyceringemisch beispielsweise durch Destillation im Hochvakuum abgetrennt werden. In der Regel kann auf diese Reinigungsoperation jedoch verzichtet werden.

Im Anschluß an die Kondensation können die Oligoglyceringemische einer **Umesterung** mit Fettsäureglyceridestern unterworfen werden.

Als **Fettsäureglyceridester** kommen Triglyceride der Formel (I) in Betracht, 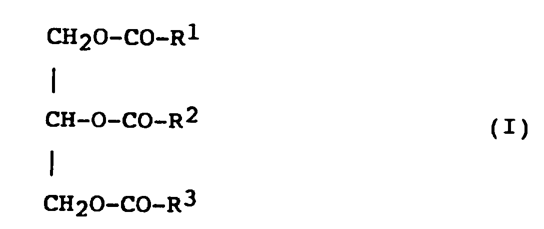 in der R¹CO für einen linearen oder verzweigten aliphatischen Acylrest mit 16 bis 24 Kohlenstoffatomen und 1 bis 5 Doppelbindungen und RCO und R³CO unabhängig voneinander für einen linearen oder verzweigten aliphatischen Acylrest mit 6 bis 24 Kohlenstoffatomen und 0 oder 1 bis 5 Doppelbindungen steht.

Das molare Verhältnis zwischen Oligoglyceringemisch und Fettsäureglyceridester kann 5 : 1 bis 1 : 5, vorzugsweise 3 : 1 bis 1 : 3 betragen. Die Umesterung kann in an sich bekannter Weise im Temperaturbereich von 200 bis 280°C durchgeführt werden. Die im Oligoglyceringemisch verbliebene Lithiumkonzentration ist ausreichend, um auch die Umesterung zu katalysieren; der Zusatz weiteren Katalysators ist somit nicht erforderlich. Die Umesterung kann vollständig, in der Regel aber partiell, beispielsweise zu 10 bis 90, insbesondere 20 bis 70 Gew.-% - bezogen auf den Fettsäureglyceridester - erfolgen.

In einer bevorzugten Ausführungsform der Erfindung können Kondensation und Umesterung nicht nacheinander, sondern in einem Schritt durchgeführt werden.

Ein weiterer Gegenstand der Erfindung betrifft die Verwendung der nach dem erfindungsgemäßen Verfahren erhältlichen Polyolverbindungen zur Herstellung von Polymeren, in denen sie zu 1 bis 90, vorzugsweise 10 bis 70 Gew.-% - bezogen auf die Polymeren - enthalten sein können.

### Gewerbliche Anwendbarkeit

Die nach dem erfindungsgemäßen Verfahren erhältlichen Polyolverbindungen eignen sich als Rohstoffe zur Herstellung von Polymeren. Sie können beispielsweise über die Hydroxylgruppen in Alkydharze einkondensiert werden und stellen Polykondensationsbausteine dar, wie sie insbesondere für die Entwicklung von Polyurethanschäumen von Wichtigkeit sind.

### Beispiele

### Beispiel 1:

In einem 2-l-Dreihalskolben mit Innenthermometer und Wasserabscheider wurden 1200 g (13 Mol) Glycerin mit 1,5 g (0,06 Mol) Lithiumhydroxid, entsprechend 0,12 Gew.-% - bezogen auf das Glycerin - versetzt. Die Reaktionsmischung wurde unter Stickstoff über einen Zeitraum von 8 h auf 260°C erhitzt, wobei eine Menge von 120 ml Kondensat abgeschieden wurde. Nach dem Abkühlen wurden ca. 1000 g des Oligoglyceringemisches als dunkle, klare Flüssigkeit erhalten, die folgende Kenndaten aufwies:

| | |
|---|---|
| Hydroxylzahl : | 1362 |
| Säurezahl : | 0,4 |
| Li-Gehalt : | 365 ppm |

### Beispiel 2:

In einem 2-l-Dreihalskolben wurde eine Mischung aus
- 994 g (1,12 Mol): Rüböl neuer Züchtung, Ölsäureanteil > 80 Gew.-% und
- 406 g (2,45 Mol): Oligoglyceringemisch aus Bsp.1
vorgelegt und unter Stickstoff über einen Zeitraum von 5 h bei 220°C umgeestert. Das Produkt separierte und wurde in einen Scheidetrichter überführt, mit dessen Hilfe man die das nichtumgesetzte Oligoglycerin enthaltende Phase (ca. 50 g) abtrennte und verwarf. Es wurden 1300 g des Umesterungsproduktes in Form einer hellbraunen, klaren Flüssigkeit erhalten, die die folgenden Kennzahlen aufwies:

| | |
|---|---|
| Hydroxylzahl : | 336 |
| Verseifungszahl : | 142 |
| Säurezahl : | 1,3 |
| Li-Gehalt : | 82 ppm |

## Patentansprüche

1. Verfahren zur Herstellung von Polyolverbindungen, **dadurch gekennzeichnet**, daß man
a) Glycerin in Gegenwart von 0,001 bis 0,1 Gew.-% - bezogen auf das Glycerin - Lithiumhydroxid oder Lithiumseifen von gegebenenfalls hydroxysubstituierten Fettsäuren mit 6 bis 22 Kohlenstoffatomen und 0, 1, 2 oder 3 Doppelbindungen kondensiert,
b) das Reaktionswasser kontinuierlich abdestilliert und
c) das erhaltene Oligoglyceringemisch mit Fettsäureglyceridestern einer Umesterung unterwirft.

2. Verfahren nach Anspruch 1, **dadurch gekennzeichnet**, daß man die Kondensationsreaktion bei Temperaturen von 200 bis 300°C durchführt.

3. Verfahren nach den Ansprüchen 1 und 2, **dadurch gekennzeichnet**, daß man als Fettsäureglyceridester Triglyceride der Formel (I), 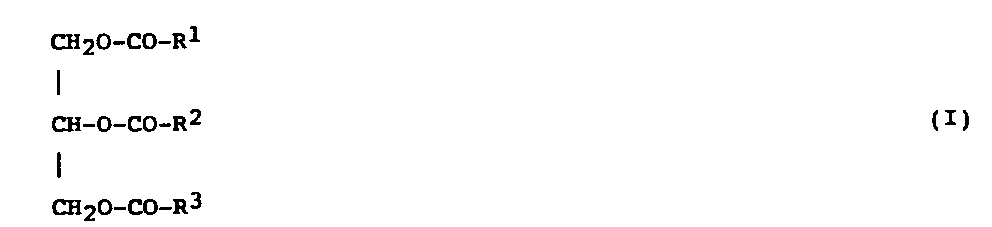 in die Umesterung einsetzt, in der R¹CO für einen linearen oder verzweigten aliphatischen Acylrest mit 16 bis 24 Kohlenstoffatomen und 1 bis 5 Doppelbindungen und RCO und R³CO unabhängig voneinander für einen linearen oder verzweigten aliphatischen Acylrest mit 6 bis 24 Kohlenstoffatomen und 0 oder 1 bis 5 Doppelbindungen steht.

4. Verfahren nach den Ansprüche 1 bis 3, **dadurch gekennzeichnet**, daß man die Oligoglyceringemische und die Fettsäureglyceridester im molaren Verhältnis von 5 : 1 bis 1 : 5 einsetzt.

5. Verfahren nach den Ansprüchen 1 bis 4, **dadurch gekennzeichnet**, daß man die Umesterung im Temperaturbereich von 200 bis 280°C durchführt.

6. Verwendung der nach dem Verfahren nach den Ansprüchen 1 bis 5 erhältlichen Polyolverbindungen zur Herstellung von Polymeren.

## Claims

1. A process for the production of polyol compounds, characterized in that
a) glycerol is condensed in the presence of 0.001 to 0.1% by weight, based on the glycerol, of lithium hydroxide or lithium soaps of optionally hydroxy-substituted fatty acids containing 6 to 22 carbon atoms and 0, 1, 2 or 3 double bonds,
b) the water of reaction is continuously distilled off and
c) the oligoglycerol mixture obtained is optionally transesterified with fatty acid glyceride esters.

2. A process as claimed in claim 1, characterized in that the condensation reaction is carried out at temperatures of 200 to 300°C.

3. A process as claimed in claims 1 and 2, characterized in that triglycerides corresponding to formula (I): 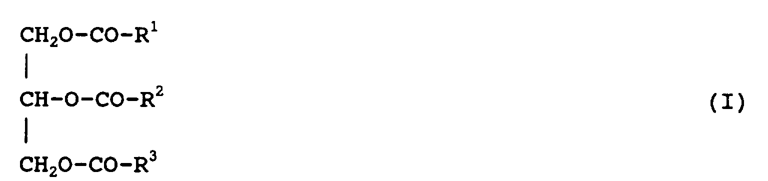 in which R¹CO is a linear or branched aliphatic acyl radical containing 16 to 24 carbon atoms and 1 to 5 double bonds and RCO and R³CO independently of one another represent a linear or branched aliphatic acyl radical containing 6 to 24 carbon atoms and 0 or 1 to 5 double bonds,
are used as the fatty acid glyceride esters.

4. A process as claimed in claims 1 to 3, characterized in that the oligoglycerol mixtures and the fatty acid glyceride esters are used in a molar ratio of 5:1 to 1:5.

5. A process as claimed in claims 1 to 4, characterized in that the transesterification is carried out at a temperature of 200 to 280°c.

6. The use of the polyol compounds obtainable by the process claimed in claims 1 to 5 for the production of polymers.

## Revendications

1. Procédé de production de composés polyols, caractérisé en ce que :
a) on condense le glycérol en présence de 0,001 à 0,1 % en poids - rapporté au glycérol - d'hydroxyde de lithium ou de savons de lithium d'acides gras éventuellement substitués par un hydroxyle, ayant de 6 à 22 atomes de carbone et 0, 1, 2 ou 3 doubles liaisons,
b) on sépare par distillation en continu l'eau de réaction et
c) on soumet le mélange d'oligoglycérols obtenu à une transestérification avec des esters de glycérides d'acide gras.

2. Procédé selon la revendication 1, caractérisé en ce que l'on exécute la réaction de condensation à des températures allant de 200 à 300°C.

3. Procédé selon les revendications 1 et 2, caractérisé en ce que l'on met en oeuvre dans la transestérification comme esters de glycérides d'acide gras, des triglycérides de formule (I) 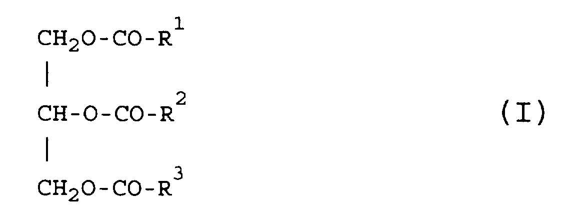 dans laquelle R¹CO représente un radical acyle aliphatique, linéaire ou ramifié, ayant de 16 à 24 atomes de carbone et de 1 à 5 doubles liaisons et RCO et R³CO, indépendamment l'un de l'autre, représentent un radical acyle aliphatique, linéaire ou ramifié, ayant de 6 à 24 atomes de carbone, et 0 ou 1 à 5 doubles liaisons.

4. Procédé selon les revendications 1 à 3, caractérisé en ce que l'on met en oeuvre les mélanges d'oligoglycérols et les esters de glycérides d'acide gras dans un rapport molaire allant de 5:1 à 1:5.

5. Procédé selon les revendications 1 à 4, caractérisé en ce que l'on effectue le transestérification dans une plage de températures allant de 200 à 280°C.

6. Utilisation des composés polyols obtenus selon le procédé conformément aux revendications 1 à 5, en vue de la production de polymères.
